# EUROPEAN PATENT SPECIFICATION

(11) **EP 1 791 588 B1**
(45) Date of publication and mention of the grant of the patent: **15.03.2017**
(21) Application number: 05797868.6
(22) Date of filing: 20.09.2005
(51) Int. Cl.: A61M 29/02, A61M 25/09, A61M 25/01

(54) **VARIABLE FLEXIBILITY WIRE GUIDE**
DRAHTFÜHRUNG MIT VARIABLER FLEXIBILITÄT
PASSE-FIL A FLEXIBILITE VARIABLE

(30) Priority: 21.09.2004 US 946416
(43) Date of publication of application: 06.06.2007
(73) Proprietor: Cook Medical Technologies LLC, Bloomington, IN 47404 (US)
(72) Inventor: OSBORNE, Thomas, A., Bloomington, IN 47401 (US); BARR, Aaron, Fishers, IN 46038 (US)
(74) Representative: Garratt, Peter Douglas
(86) International application number: PCT/US2005/033722
(87) International publication number: WO 2006/034302

(56) References cited:
- EP-A- 0 382 974
- EP-A- 0 806 219
- EP-A- 1 287 846
- WO-A-2004/018031
- US-A1- 2004 167 438
- US-B1- 6 694 595

## Description

### BACKGROUND

### 1. Field of the Invention

The present invention generally relates to a medical surgical device and specifically a wire guide for percutaneous placement providing variable flexibility along its length.

### 2. Description of Related Art

Wire guides are widely used throughout the medical industry. Wire guides are used for advancing intraluminal devices such as stent delivery catheters, balloon dilation catheters, atherectomy catheters, and the like within body lumens. Typically, the wire guide is positioned inside the inner lumen of an introducer catheter. The wire guide is advanced out of the distal end of the introducer catheter into the patient until the distal end of the wire guide reaches the location where the interventional procedure is to be performed. After the wire guide is inserted, another device such as a stent and stent delivery catheter is advanced over the previously introduced wire guide into the patient until the stent delivery catheter is in the desired location. After the stent has been delivered, the stent delivery catheter can then be removed from a patient by retracting the stent delivery catheter back over the wire guide. The wire guide may be left in place after the procedure is completed to ensure easy access if it is required. Conventional wire guides include an elongated wire core with one or more tapered sections near the distal end to increase flexibility. Generally, a flexible body such as a helical coil or tubular body is disposed about the wire core. The wire core is secured to the flexible body at the distal end by soldering, brazing or welding which forms a rounded distal tip. In addition, a torquing means is provided on the proximal end of the core member to rotate, and thereby steer a wire guide having a curved tip, as it is being advanced through a patient's vascular system.

A major requirement for wire guides and other intraluminal guiding members, is that they have sufficient stiffness to be pushed through the patient's vascular system or other body lumen without kinking. However, they must also be flexible enough to pass through the tortuous passageways without damaging the blood vessel or any other body lumen through which they are advanced. Efforts have been made to improve both the strength and the flexibility of wire guides in order to make them more suitable for their intended uses, but these two properties tend to be diametrically opposed to one another in that an increase in one usually involves a decrease in the other.

For certain procedures, such as when delivering stents around challenging take-off, tortuosities, or severe angulation, substantially more support and/or vessel straightening is frequently needed from the wire guide. Wire guides have been commercially available for such procedures which provide improved support over conventional wire guides. However, such wire guides are not very steerable and in some instances are so stiff they can damage vessel linings when being advanced.

In other instances, extreme flexibility is required as well. For example, when branched or looped stents are to be delivered to a branched vascular region, it is beneficial to insert the wire guide from the branch where a stent is to be located. However, the stent may need to be introduced and guided from a separate branch. In this situation, the wire guide is inserted into the patient's vascular system near the desired stent location and a grasping device is inserted in the branch from which the stent will be introduced. The wire guide may be advanced back along the branch to provide the grasping device access to the distal end of the wire guide. However, the wire guide should be extremely flexible to allow grasping and manipulation of the wire guide without damaging the tissue around the bifurcation formed by the luminal branch. Further, the wire guide should be extremely kink resistant to avoid damaging the wire guide as it is grasped. After the wire guide is retrieved by the grasping device, the stent may be delivered over the wire guide to the desired location. However, available wire guides are not designed to provide the flexibility required to cross up and over the bifurcation of the luminal branch and yet also provide the stiffness required to aid in the insertion of the stent.

In view of the above, it is apparent that there exists a need for an improved design for a wire guide.

### SUMMARY OF THE INVENTION

In satisfying the above need, as well as, overcoming the enumerated drawbacks and other limitations of the related art, the present invention provides a wire guide having a wire core and a braided sheath. The braided sheath is attached to a first end of the wire core and serves as a flexible pulling section. The braided sheath is woven of a plurality of strands and may be made of various material based on the application, such as stainless steel, a shape memory alloy, or a radiopaque material. The wire guide also has a flexible tip opposite the flexible pulling section. A stiff section is provided between the flexible tip and the flexible pulling section to allow manipulation of the wire guide through a body lumen.

Toward the first end of the wire core, a tapered section is provided to increase flexibility of the wire guide over the flexible pulling section. The braided sheath is received over the wire core and is attached to the wire core by solder or adhesive. In addition, a shoulder is provided in the wire core facilitating a smooth transition from the wire core to the braided sheath. The braided sheath extends from the shoulder beyond the end of the wire core, thereby forming the flexible pulling section.

The flexible tip is provided opposite the flexible pulling section, near the second end of the wire core and includes a tapered section reducing the diameter of the wire core toward the flexible tip. A coil member is disposed about the second end and attached to the wire core. A sleeve, such as, a polyurethane layer surrounds the wire core and the braided sheath to improve kink resistance and guidablity of the wire guide. In addition, a lubricous coating is provided over the sleeve to improve the ease of advancement of the wire guide through the patient's vascular system. The lubricous coating may be a hydrophilic coating and may be omitted from the flexible pulling section to improve graspability of the braided sheath.

It is an object of the present invention to provide a wire guide with a flexible pulling section that can be easily grasped from an opposite branch while providing enough stiffness to aid in location of the wire guide and placement of a medical device over the wire guide.

In one aspect of the invention, a wire guide for introducing medical devices into a patient is as defined in claim 1.

In another aspect of the invention, the braided member is soldered to the first end of the core member.

In another aspect of the invention, an adhesive configured to attach the braided member to the first end of the core member.

In another aspect of the invention, a lubricious coating surrounds the core member.

In another aspect of the invention, the lubricious coating is a hydrophilic coating.

In another aspect of the invention, the core member includes a first tapered section proximal the braided member, the first tapered section being configured to increase flexibility toward the first end of the core member.

In another aspect of the invention, the core member includes a second tapered section, configured to increase flexibility toward the second end of the core member.

In another aspect of the invention, the core member includes a stiff section between the first tapered section and the second tapered section.

In another aspect of the invention, the stiff section is between about 50 to 200 cm in length.

In another aspect of the invention, a distance from the second tapered section to the second end of the core member is between about 3 and 5 cm.

In another aspect of the invention, a distance including the first tapered section and extending to an end of the braided member is between about 40 and 80 cm.

In another aspect of the invention, the first tapered section is between about 5 and 15 cm.

In another aspect of the invention, a distance from the first tapered section to the first end of the core member is between about 5 and 15 cm.

In another aspect of the invention, the braided member extends beyond the first end of the core member by a distance of between about 30 and 50 cm.

In another aspect of the invention, a coil member disposed about the second end of the core member.

In another aspect of the invention, the coil member is a platinum coil.

In another aspect of the invention, the coil member is soldered to the core member.

In another aspect of the invention, the core member is comprised of a shape memory alloy.

In another aspect of the invention, the core member is comprised of Nitinol.

In another aspect of the invention, the plurality of strands are comprised of stainless steel.

In yet another aspect of the invention, the plurality of strands are comprised of a shape memory alloy.

In yet another aspect of the invention, the plurality of strands are comprised of Nitinol.

In yet another aspect of the invention, the plurality of strands are comprised of a radiopaque material.

In yet another aspect of the invention, a coil member extending along the length of the core member.

In yet another aspect of the invention, a braided member is attached to the coil member.

In yet another aspect of the invention, the wire guide comprises a sleeve surrounding the core member and the braided member.

In yet another aspect of the invention, the sleeve is a polyurethane layer surrounding the core member and braided member.

In yet another aspect of the invention, the braided member is located about the core member.

In yet another aspect of the invention, the braided member has a braided member diameter not substantially greater than the core member diameter of the core member.

Further objects, features and advantages of this invention will become readily apparent to persons skilled in the art after a review of the following description, with reference to the drawings and claims that are appended to and form a part of this specification.

### BRIEF DESCRIPTION OF THE DRAWINGS

FIG. 1 is a partial sectional view of a wire guide embodying the principles of the present invention;
FIG. 2 is a cross sectional view of an aneurysm illustrating the insertion of a stent graft delivery system and a wire guide embodying the principles of the present invention;
FIG. 3 is a cross sectional view of an aneurysm illustrating the stent graft delivery system and the wire guide being advanced therefrom;
FIG. 4 is a cross sectional view of an aneurysm illustrating a snare pulling the wire guide across the bifurcation between the femoral branches;
FIG. 5 is a cross sectional view of an aneurysm illustrating the side branch stent graft being partially unsheathed;
FIG. 6 is a cross sectional view of an aneurysm illustrating the delivery sheath and dilator for the side branch extension stent graft being introduced over the wire guide;
FIG. 7 is a cross sectional view of an aneurysm illustrating the delivery sheath for the side branch extension stent graft being advanced through the side branch stent graft;
FIG. 8 is a cross sectional view of an aneurysm illustrating the wire guide being pulled out of the side branch stent graft delivery sheath to free the arm of the side branch stent graft;
FIG. 9 is a cross sectional view of an aneurysm illustrating the deployment of the side branch stent graft;
FIG. 10 is a cross sectional view of an aneurysm illustrating the deployment of the side branch extension stent graft;
FIG. 11 is a cross sectional view of an aneurysm illustrating the deployment of the main body stent graft over the wire guide;
FIG. 12 is a cross sectional view of an aneurysm illustrating a completed stent graft installation with all delivery systems removed;
FIG. 13 is a partial sectional view of a wire guide having a coil member along its length and embodying the principles of the present invention.

### DETAILED DESCRIPTION OF THE INVENTION

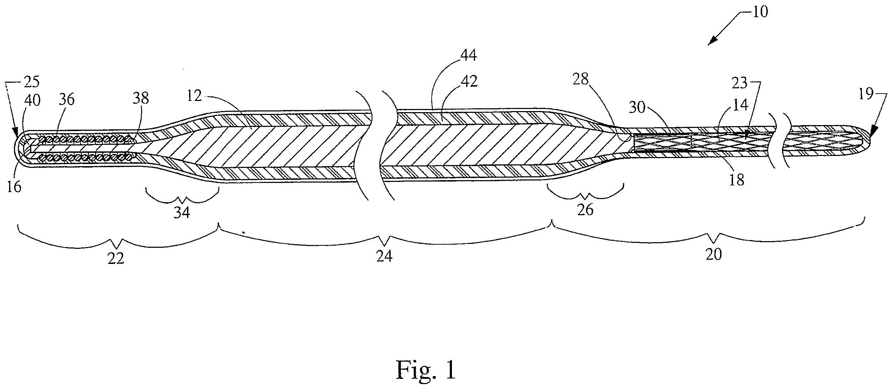

Referring now to Figure 1, a wire guide embodying the principles of the present invention is illustrated therein and designated at 10. The wire guide 10 includes a core member, such as, a wire core 12 and a braided member, such as, braided sheath 14. The braided sheath 14 and a portion of the wire core 12 cooperate to form a flexible pulling section 20 near a first end 19 of the wire guide 10. Opposite the flexible pulling section 20 is a flexible tip section 22 located near a second end 25 of the wire guide 10. Between the flexible pulling section 20 and the flexible tip section 22, is a stiff middle section 24.

Each of the three sections 20, 22, and 24 are particularly beneficial for inserting a stent around a branched or looped body lumen. Sometimes, it is beneficial to insert the wire guide 10 from the branch where the stent is to be located, however, the stent may need to be introduced and guided from a separate branch. The first end 19 of the wire guide 10 is inserted into the patient's vascular system near the desired stent location. Similarly, a grasping device can be inserted in another branch from which the stent will be introduced. The wire guide 10 is advanced back along the branch to provide the grasping device access to the first end 19 of the wire guide 10. However, the wire guide 10 must be extremely flexible to allow grasping and manipulation of the first end 19 without damaging the tissue around the bifurcation formed by the luminal branch. Accordingly, the braided sheath 14 provides the needed flexibility in the flexible pulling section 20 of the wire guide 10. The flexible pulling section 20 may be retrieved by the grasping device through the entry in other branch. The flexible tip section 22 is pulled into the patient and the stiff middle section 24 is used to manipulate the flexible tip section 22 to a location of interest. The described configuration provides access for other devices to be advanced along the wire guide 10 to the location of interest.

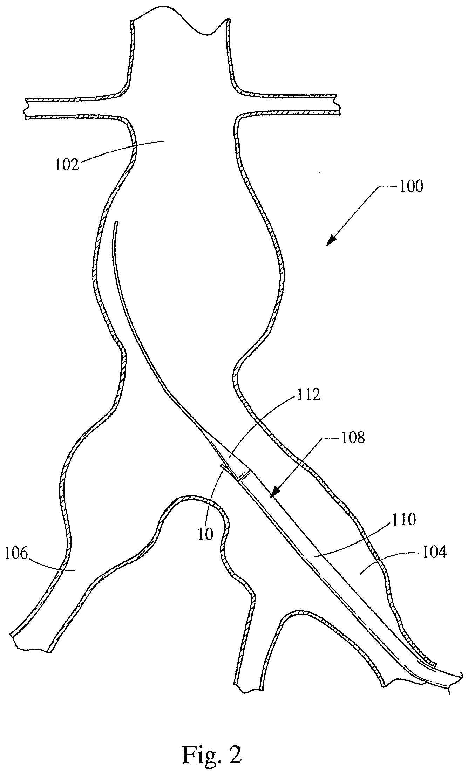

A detailed example of such a procedure is illustrated in Figures 2-12. An arterial aneurysm 100 extends from the aorta 102 into a first femoral branch 104 and a second femoral branch 106.

Figure 2 shows the side branch stent graft and delivery system 108 inserted and positioned near the target side branch artery. The wire guide 10 of this invention is shown protruding slightly from between the delivery sheath 110 and the inner dilator 112. The dilator 112 has a small groove to accommodate the wire guide 10.

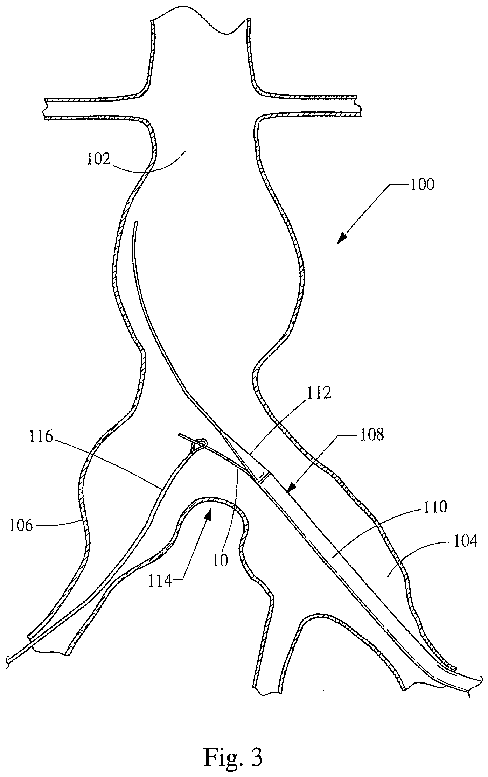

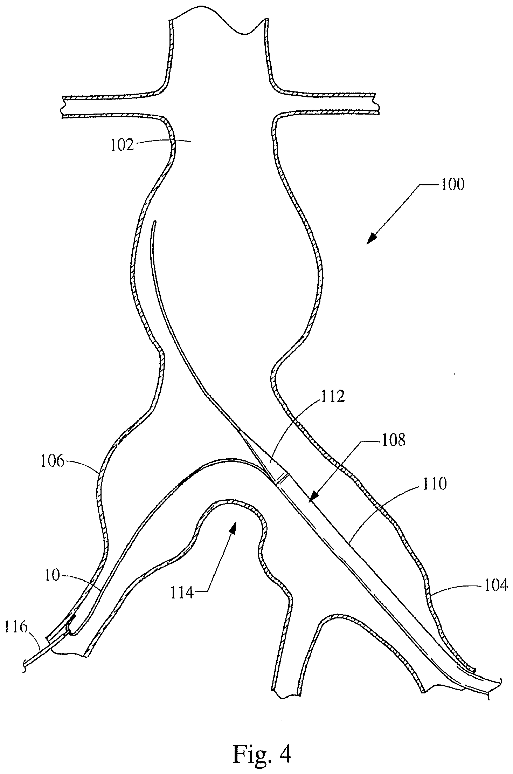

Now referring to Figures 3 and 4, the wire guide 10 must be snared, pulled to the opposite side entry site and pulled out of the entry site to a point external to the patient. Figure 3 shows the wire guide 10 of this invention advanced a few centimeters to provide enough length of wire so that the snare 116 can securely capture and pull the wire guide 10 over the bifurcation 114 and out the snare entry site. In this maneuver, the end of the wire guide 10 is folded, or doubled over as it is pulled by the snare 116 through the artery and out the entry site of the snare 116. This requires that the wire guide 10 be very flexible in this section so as to not traumatize the artery wall while making a very small radius fold. Further, the wire guide 10 must be strong enough to withstand the tensile forces of the pulling through process and not be permanently kinked or deformed such that, the side branch extension delivery system can be loaded onto the wire guide 10 once the end has been pulled out.

Ordinary wire guide construction is not suitable for these requirements. The small, "safety" wires used in conventional flexible tip wire guides do not have suitable tensile strength to insure that the wire will not break allowing the coil to unravel or stretch, thereby becoming unusable. The use of the fine wire braid as a safety wire increases the tensile strength of the "safety" wire and does not add appreciable stiffness. Typical safety wires are small round or rectangular wires, .003 to .005 in. (0,0762 to 0,1270 mm) diameter or .002 by .004 in. (0,0508 by 0,1016 mm) rectangular with tensile strengths in the range of 2 to 10 pounds (0,907 to 1,814 kg) pull strength. The multiple fine wire braid material can have a tensile strength from 10 to 25 pounds (4,536 to 9,072 kg) pull strength.

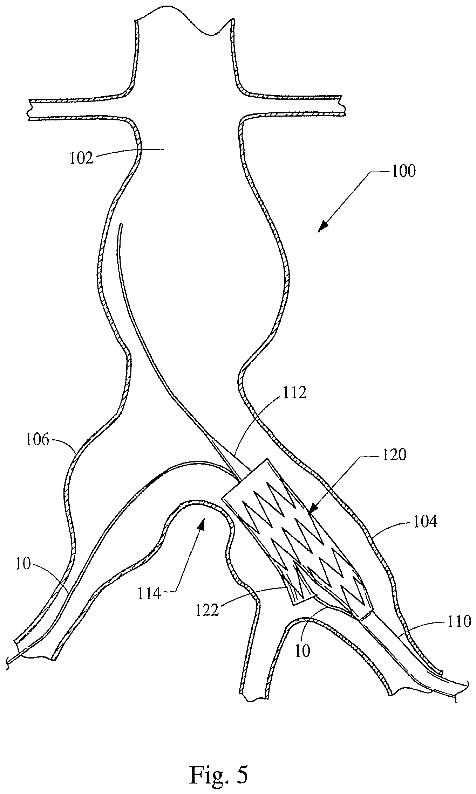

Now referring to Figures 4 and 5, the wire guide 10 used in this procedure must be pulled across the bifurcation between the femoral arteries 106, 104 and the aorta 102. Figure 4 shows the wire guide 10 snared and pulled over the bifurcation 114 and toward the (entry site for the snare 116) on the opposite side. The artery wall around the bifurcation 114 is very thin and fragile due to the aneurismal disease that the stent grafts are attempting to repair. Therefore, the body of the wire guide 10 needs to be smooth and slippery. Typical wire guides are coils with stiffening central cores or mandrels. The surface of a coil type wire guide is "bumpy" due to the successive coils along the length of the wire guide. Pulling this type of surface across tissue can result in abrasion of the diseased or damaged tissue, increasing the risk of aneurism rupture during the repair procedure. The wire guide 10 of this invention uses a smooth body portion to protect the artery wall in the area of the bifurcation. The smooth, non-traumatic surface can be achieved by eliminating the outer coil portion and increasing the diameter of the coil portion an appropriate amount, then coating the body portion with a soft polymer material such as polyurethane, then coating the polymer with a lubricious, hydrophilic coating to lower the coefficient of friction between the artery wall the body of the wire guide 10. In addition, the wire guide 10 must be stiff enough to provide guidance or direction for the side branch extension stent graft delivery system. Normal percutaneous entry wire guides are not stiff enough to control and deflect a device as bulky and stiff as a stent graft delivery system.

Figure 5 shows the wire guide 10 of this invention pulled across the bifurcation 114 and out the snare entry site on the opposite side. The side branch stent graft 120 has been partially unsheathed, exposing the short side branch leg of the stent graft 120. The wire guide 10 of this invention still passes through the side branch stent graft 120 through the short arm 122 and back into the sheath 110.

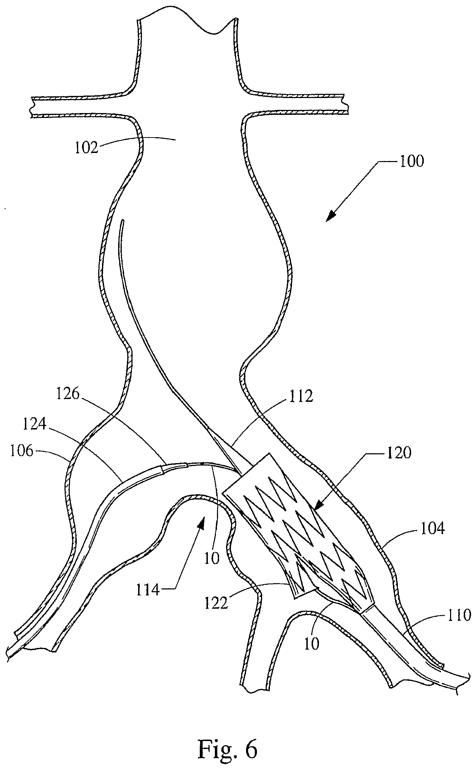

Figure 6 shows the delivery sheath 124 and dilator 126 for the side branch extension stent graft being introduced and advanced over the wire guide 10 of this invention from the opposite side.

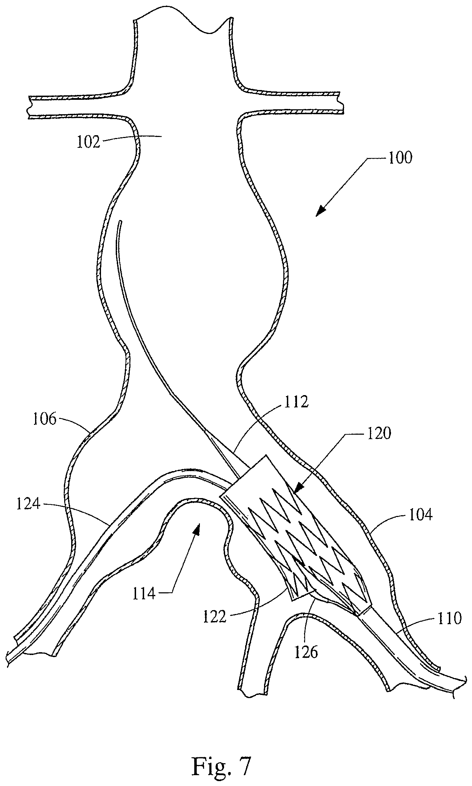

Figure 7 shows the delivery sheath 124 and dilator 126 for the side branch extension stent graft being advanced through the side branch stent graft 120 all the way to the point where the wire guide 10 of this invention enters the delivery sheath 110 of the side branch stent graft 120.

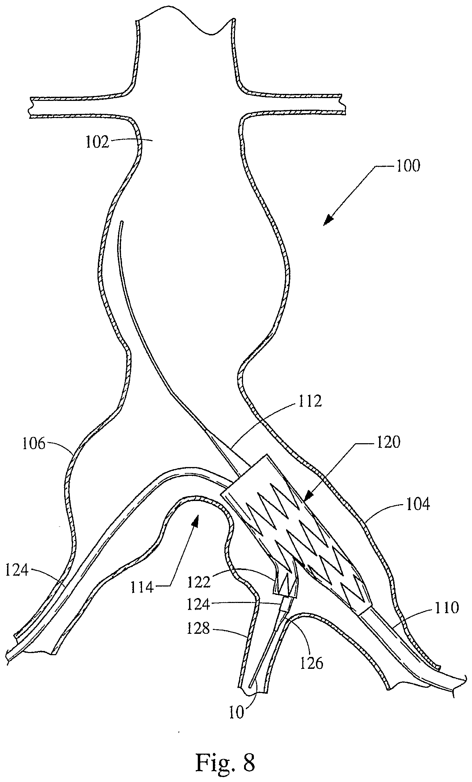

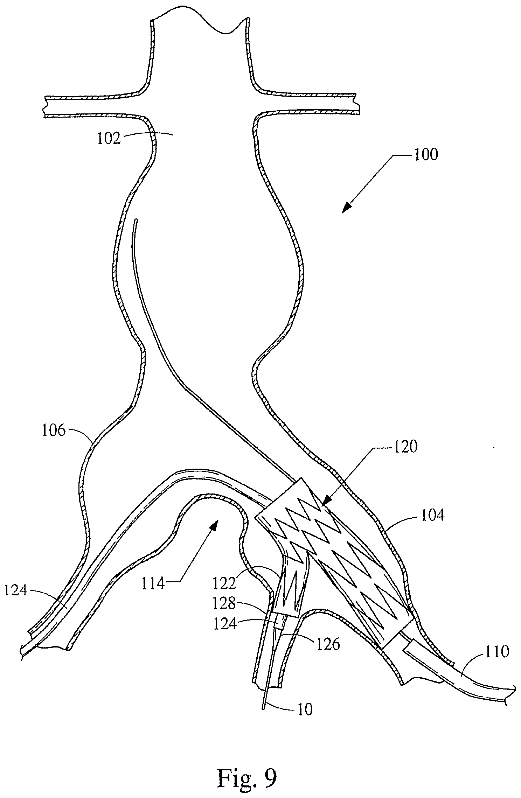

Now referring to Figures 8 and 9, the wire guide 10 of this invention must also have a flexible portion at the opposite end located in the target branch 104. This is the end of the wire that is used to enter the target side branch artery 128 where the extension stent graft 130 is to be placed. If the end of the wire guide 120 that is being advanced into the side branch artery is stiff, the physician will not be able to direct the wire into the desired artery and the end of the wire would be traumatic and damage artery wall as it is advanced along the artery.

Figure 8 shows the wire guide 10 of this invention pulled out through the side branch extension stent graft delivery system 108 until the opposite end of the wire guide 10 exits the distal end of the side branch stent graft delivery sheath 110, freeing the short arm 122 of the stent graft 120 and allowing the wire guide 10 of this invention to be advanced with the delivery sheath 124 through the short arm extension stent graft into the target side branch artery 128.

Figure 9 shows the side branch stent graft delivery sheath 110 withdrawn, completing the deployment of the side branch stent graft 120.

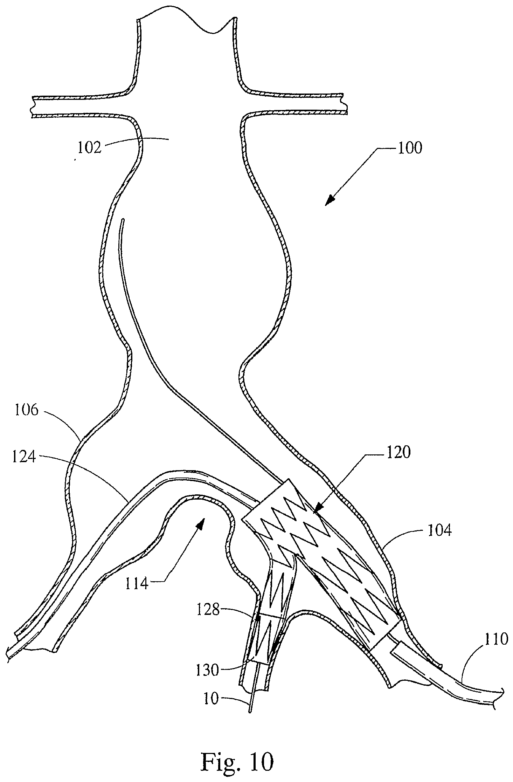

Figure 10 shows the short arm extension stent graft 130 delivered and deployed over the wire guide 10 of this invention. The wire guide 10 of this invention and the extension stent graft delivery sheath 124 are still in place.

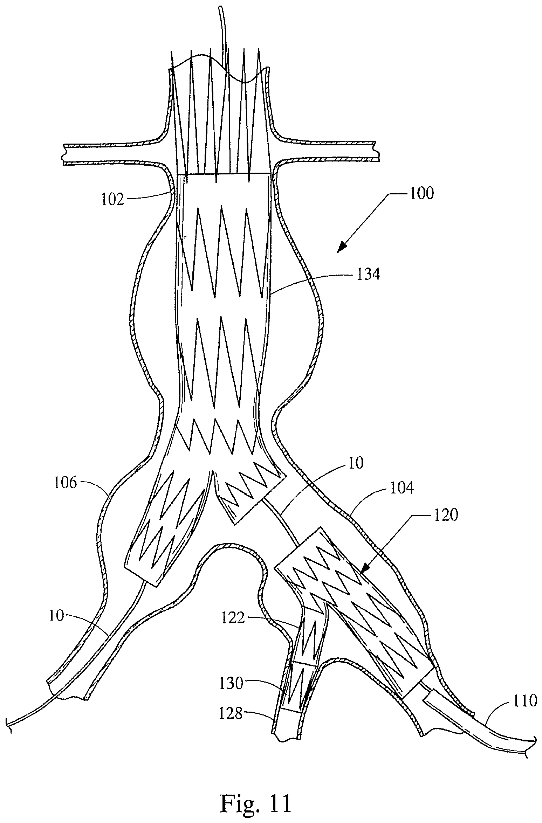

Figure 11 shows the short arm extension stent graft delivery sheath 124 withdrawn and removed. The wire guide 10 of this invention has been withdrawn from across the bifurcation 114 and used for the delivery and deployment of the main body stent graft 134.

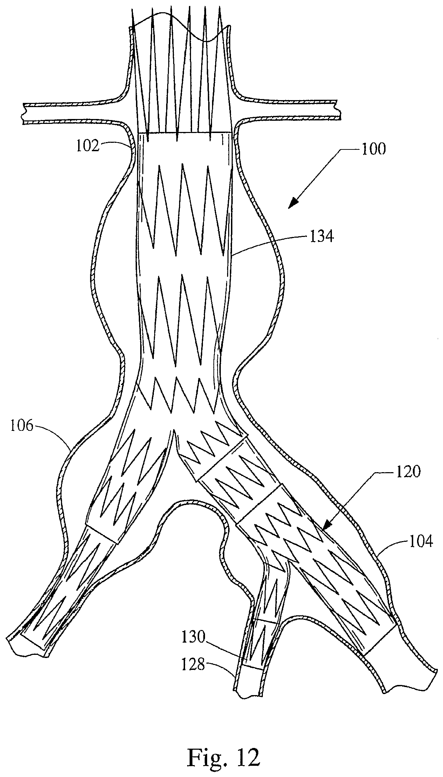

Figure 12 shows the completed stent graft installation with all stent grafts in place and delivery systems removed.

Referring again to Figure 1, additional flexibility is provided in the flexible pulling section 20 by a tapered section 24 that reduces the diameter of the wire core 12 towards a first end 18 of the wire core 12. In addition, the braided sheath 14 is attached to and extends from the first end 18 of the wire core 12. Preferably, the braided sheath 14 is received over and around the first end 18 and is attached to the wire core 12 by a bond 30 of solder or adhesive. A shoulder 28 is provided allowing the braided sheath 14 to seat against the shoulder 28. The radial height of the shoulder 28 is about the thickness of the braided sheath 14 thereby providing a smooth transition from the wire core 12 to the braided sheath 14 surrounding the first end 18. Further, the braided sheath 14 extends from the shoulder 28 beyond the first end 18 of the wire core 12.

The braided sheath 14 provides increased flexibility and kink resistance in combination with strength and graspability to provide benefits over other more common methods of providing wire guide flexibility. The braided sheath 14 is constructed of a plurality of strands 23 interwoven to provide strength to the braided sheath 14. The strands 23 are wrapped in a clockwise and counterclockwise direction, with strands weaving in and out of other strands. The density, thickness, or material of the strands may be varied to increase or decrease the flexibility along the braided sheath. The strands 23 are comprised of stainless steel or other common materials. Alternatively, the strands 23 may be comprised of Nitinol to provide increased control over the flexibility of the braid or a radiopaque material to provide increased visibility during grasping of the flexible pulling section 20.

The stiff middle section 24 allows the physician to direct the second end 25 of the wire guide 10 into sub-branches or further down the body lumen into which it was inserted. To provide improved control over flexibility of the wire guide 10, the wire core 12 is comprised of a shaped memory alloy, such as Nitinol. Alternatively, the wire core 12 may be constructed of commonly used wire guide material such as stainless steel.

To provide protection for the surrounding tissue as the second end 25 is being directed, the flexible tip section 22 is provided. The flexible tip section 22 includes a second tapered section 34. The second tapered section 34 reduces the diameter of the wire core 12 toward the second end 25 of the wire guide 10 thereby providing increased flexibility. A coil member 36 is disposed about the wire core 12. The coil member 36 is attached to the wire core 12 near the second tapered section 34 by solder joint 38 and at a second end 16 of the wire core 12 by a solder joint 40 that is formed into a rounded tip. The coil member 36 acts to control the flexibility of the wire core 12 along the flexible tip section 22. The coil 36 member is made of a radiopaque material, such as, platinum. Using a radiopaque material, allows for better visibility during manipulation of the wire guide 10.

The proportions of the flexible pulling section 20, stiff middle section 24, and flexible tip section 22 are also notable aspects of the wire guide 10. The wire guide 10 must be long and stiff enough to aid in the insertion of a stent, while being flexible enough and providing a long enough flexible pulling section 20 to allow the wire guide 10 to cross up and over the bifurcation of the branch, aiding in retrieval of the wire guide 10. Accordingly, for the delivery of a stent for treating aortic abdominal aneurism, the stiff middle section 24 is between about 50 and 200 cm in length, preferably about 100 cm, and having a core diameter of about 0.035 mm.

The flexible pulling section 20 includes the first tapered section 26 and extends along the length of the braided sheath 14. The flexible pulling section 20 is between about 40 and 80 cm, preferably about 60 cm in length. The braided sheath 14 has a similar diameter to the core diameter, more specifically the braided sheath 14 may have a diameter substantially the same or less than the core member 12. Further, the first tapered section 26 is between about 5-15 cm in length, preferably between 8-10 cm; the distance from the first tapered section 26 to the distal end 18 of the wire core 12 is between about 5-15 cm, preferably about 10 cm; and the braided sheath 14 extends beyond the first end 18 of the wire core 12 by between about 30-50 cm, preferably about 40 cm. In addition the flexible tip 22 from the second tapered section 34 to the second end 16 of the wire core 12 is between about 3 and 5 cm in length. Although, these dimensions provide advantages for the above mentioned application, differing lengths are contemplated and may be more suitable for other applications. Further, certain aspects of the drawings such as the tapers may be exaggerated for illustrative purposes.

A sleeve 42 is disposed about the wire core 12 and the braided sheath 14 to provide to provide a smooth contiguous surface, so as not to damage the diseased tissue as the wire guide 10 is pulled over the bifurcation of the luminal branch. The sleeve 42 may be made of polyurethane or other commonly used sleeve materials to improve the performance of wire guides. In addition, a lubricous coating 44 is applied over the sleeve section 42. The lubricous coating 44 may be a hydrophilic coating to reduce surface friction, thereby improving the ease with which the wire guide 10 may be advanced through the body lumen. The hydrophilic coating may encompass the entire length of the wire guide 10, or alternatively, may encompass the wire core 12 but not the flexible pulling section 20 to provide improved graspability of the braided sheath 14.

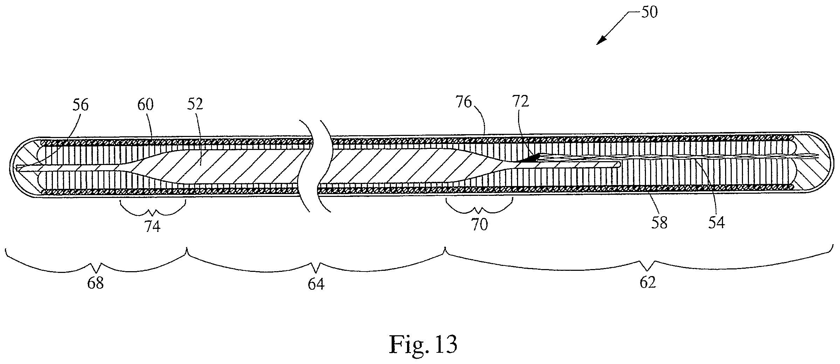

Now referring to Figure 13, another embodiment of a wire guide 50 is provided having a wire core 52, braided sheath 54, and a coil member 60. The coil member 60 is attached to and disposed about the wire core 52 and braided sheath 54. Similar to the previous embodiment, the wire guide 50 has a flexible pulling section 62, a stiff middle section 64, and a flexible tip section 68.

The flexible pulling section 62 is formed by the wire core 52, the braided sheath 54, and the coil member 60. To provide the flexible pulling section 62, a tapered section 70 reduces the diameter of the wire core 52 towards a first end 58 providing additional flexibility. The braided sheath 54 is attached to the wire core 52 near the first end 58. Preferably, the braided sheath 54 is attached to the wire core 52 by a bond 72 of solder or adhesive. The braided sheath 54 is attached to the coil member 60 creating a mechanical link between the wire core 52 and the coil member 60. The braided sheath 54 may be attached to the coil member 60 by soldering, or other common attachment methods. The mechanical link between the wire core 52 and the coil member 60 provides tension to the coil member 60, while the flexibility of the braided sheath 54 results in increased flexibility along the flexible pulling section 62.

The stiff middle section 64 allows the physician to guide a flexible tip section 68 into sub-branches or further down the body lumen into which the wire guide 50 was inserted. To provide improved control over flexibility of the wire guide 50, the wire core 52 is comprised of a shaped memory alloy, such as, Nitinol. Alternatively, the wire core 52 may be constructed of commonly used wire guide material such as stainless steel.

To provide protection to vascular tissue as the flexible tip section 68 is being directed, the flexible tip section 68 includes a second tapered section 74. The second tapered section 74 reduces the diameter of the wire core 52 toward the proximal end 56 thereby providing increased flexibility. The wire core 52 is attached to the coil member 60 at a second end 56 of the wire core 52. The second end 56 may be attached to the coil member 60 by soldering or other common attachment methods.

Further, a friction reducing layer 76 is disposed about the coil member 60. The friction reducing layer 76 may be a sleeve or coating, such as, a Teflon coating to increase the ease, with which, the wire guide 50 may be advance through the patient's vascular system. In addition, the friction reducing layer 76 serves to provide a smooth outer diameter of the wire guide 50, so as not to damage the diseased tissue as the wire guide 50 is pulled over the bifurcation of the luminal branch.

As a person skilled in the art will readily appreciate, the above description is meant as an illustration of implementation of the principles this invention. This description is not intended to limit the scope or application of this invention in that the invention is susceptible to modification, variation and change, without departing from scope of this invention, as defined in the following claims.

## Claims

1. A wire guide (10) for introducing medical devices into a patient, the wire guide comprising:
a core member (12) having a first and second end; and
a braided member (14) woven of a plurality of strands (23), the braided member (14) being affixed to the core member (12), whereby
the core member includes a shoulder (28) and a height of the shoulder (28) is about the thickness of the braided member (14) to thereby provide a smooth transition from the core member (12) to the braided member (14); and
the braided member (14) is located about a portion of the core member (12), **characterized in that** the braided member 14 extends from the shoulder (28) beyond the first end of the core member (12) to provide a flexible end section (20) of the wire guide (10).

2. The wire guide of claim 1, wherein the braided member (14) is soldered to the first end of the core member (12).

3. The wire guide of claim 1, further comprising an adhesive configured to attach the braided member (14) to the first end of the core member (12).

4. The wire guide of any of the previous claims, further comprising a lubricious coating (44) surrounding the core member (12).

5. The wire guide of claim 4, wherein the lubricious coating (44) is a hydrophilic coating.

6. The wire guide of any of the previous claims, wherein the core member (12) includes a first tapered section (26) proximal the braided member (14), the first tapered section (26) being configured to increase flexibility of the core member (12) toward the first end of the core member (12).

7. The wire guide of claim 6, wherein the core member (12) includes a second tapered section (34), configured to increase flexibility of the core member (12) toward the second end of the core member.

8. The wire guide of claim 7, wherein the core member includes a stiff section (24) between the first tapered section (26) and the second tapered section (34).

9. The wire guide of claim 8, wherein the stiff section (24) is between about 50 to 200 cm in length.

10. The wire guide of claim 7, wherein a distance from the second tapered section (34) to the second end of the core member (12) is between about 3 and 5 cm.

11. The wire guide of claims 6-10, wherein the first tapered section (26) and the braided member (14) comprise a total length of about 40 to 80 cm.

12. The wire guide of claims 6-11, wherein the first tapered section (26) comprises a length of about 5 to 15 cm.

13. The wire guide of claims 6-12, wherein a distance from the first tapered section (26) to the first end of the core member (12) is between about 5 and 15 cm.

14. The wire guide of any of the previous claims, wherein the braided member (14) extends beyond the first end of the core member (12) by a distance of between about 30 and 50 cm.

15. The wire guide of claims 1-14, further comprising a coil member (60) extending along the length of the core member (12).

16. The wire guide of claim 15, wherein braided member (14) is attached to the coil member (60).

17. The wire guide of any of the previous claims, further comprising a coil member (60) disposed about the second end of the core member (12).

18. The wire guide of claim 17, wherein the coil member (60) is a platinum coil.

19. The wire guide of claim 17, wherein the coil member (20) is soldered to the core member (12).

20. The wire guide of any of the previous claims, wherein the core member (12) is comprised of a shape memory alloy.

21. The wire guide of claim 20, wherein the core member (12) is comprised of Nitinol.

22. The wire guide of any of the previous claims, wherein the plurality of strands (23) are comprised of stainless steel.

23. The wire guide of claims 1-21, wherein the plurality of strands (23) are comprised of a shape memory alloy.

24. The wire guide of claim 23, wherein the plurality of strands (23) are comprised of Nitinol.

25. The wire guide of claims 1-21, wherein the plurality of strands (23) are comprised of a radiopaque material.

26. The wire guide according to any of the previous claims, further comprising a sleeve (42) surrounding the core member (12) and the braided member (14).

27. The wire guide of claim 26, wherein the sleeve (42) is a polyurethane layer surrounding the core (12) member and braided member (14).

28. The wire guide according to any of the previous claims, wherein the braided member (14) is located about the core member (12).

29. The wire guide according to any of the previous claims, wherein the braided member (14) has a braided member diameter not substantially greater than a core member diameter of the core member (12).

## Patentansprüche

1. Drahtführung (10) zum Einführen von medizinischen Vorrichtungen in einen Patienten, wobei die Drahtführung umfasst:
ein Kernelement (12) mit einem ersten und zweiten Ende; und
ein geflochtenes Element (14), das aus einer Vielzahl von Strängen (23) gewebt ist, wobei das geflochtene Element (14) an dem Kernelement (12) fixiert ist, wobei
das Kernelement eine Schulter (28) einschließt, und eine Höhe der Schulter (28) etwa der Dicke des geflochtenen Elements (14) entspricht, um dadurch einen glatten Übergang von dem Kernelement (12) zu dem geflochtenen Element (14) bereitzustellen; und
sich das geflochtene Element (14) um einen Abschnitt des Kernelements (12) befindet, **dadurch gekennzeichnet, dass** sich das geflochtene Element (14) von der Schulter (28) über das erste Ende des Kernelements (12) hinaus erstreckt, um einen flexiblen Endabschnitt (20) der Drahtführung (10) bereitzustellen.

2. Drahtführung nach Anspruch 1, wobei das geflochtene Element (14) an das erste Ende des Kernelements (12) gelötet ist.

3. Drahtführung nach Anspruch 1, ferner umfassend einen Kleber, der ausgelegt ist, um das geflochtene Element (14) an dem ersten Ende des Kernelements (12) zu befestigen.

4. Drahtführung nach einem der vorhergehenden Ansprüche, ferner umfassend eine schmierende Beschichtung (44), die das Kernelement (12) umgibt.

5. Drahtführung nach Anspruch 4, wobei die schmierende Beschichtung (44) eine hydrophile Beschichtung ist.

6. Drahtführung nach einem der vorhergehenden Ansprüche, wobei das Kernelement (12) einen ersten sich verjüngenden Abschnitt (26) proximal des geflochtenen Elements (14) einschließt, wobei der erste sich verjüngende Abschnitt (26) ausgelegt ist, um die Flexibilität des Kernelements (12) zu dem ersten Ende des Kernelements (12) hin zu erhöhen.

7. Drahtführung nach Anspruch 6, wobei das Kernelement (12) einen zweiten sich verjüngenden Abschnitt (34) einschließt, der ausgelegt ist, um die Flexibilität des Kernelements (12) zu dem zweiten Ende des Kernelements hin zu erhöhen.

8. Drahtführung nach Anspruch 7, wobei das Kernelement einen steifen Abschnitt (24) zwischen dem ersten sich verjüngenden Abschnitt (26) und dem zweiten sich verjüngenden Abschnitt (34) einschließt.

9. Drahtführung nach Anspruch 8, wobei der steife Abschnitt (24) eine Länge von etwa 50 bis 200 cm aufweist.

10. Drahtführung nach Anspruch 7, wobei eine Distanz von dem zweiten sich verjüngenden Abschnitt (34) zu dem zweiten Ende des Kernelements (12) zwischen etwa 3 und 5 cm liegt.

11. Drahtführung nach den Ansprüchen 6 bis 10, wobei der erste sich verjüngende Abschnitt (26) und das geflochtene Element (14) eine Gesamtlänge von etwa 40 bis 80 cm umfassen.

12. Drahtführung nach den Ansprüchen 6 bis 11, wobei der erste sich verjüngende Abschnitt (26) eine Länge von etwa 5 bis 15 cm umfasst.

13. Drahtführung nach den Ansprüchen 6 bis 12, wobei eine Distanz von dem ersten sich verjüngenden Abschnitt (26) zu dem ersten Ende des Kernelements (12) zwischen etwa 5 und 15 cm liegt.

14. Drahtführung nach einem der vorhergehenden Ansprüche, wobei sich das geflochtene Element (14) um eine Distanz zwischen etwa 30 und 50 cm über das erste Ende des Kernelements (12) hinaus erstreckt.

15. Drahtführung nach den Ansprüchen 1 bis 14, ferner umfassend ein Spiralelement (60), das sich entlang der Länge des Kernelements (12) erstreckt.

16. Drahtführung nach Anspruch 15, wobei das geflochtene Element (14) an dem Spiralelement (60) befestigt ist.

17. Drahtführung nach einem der vorhergehenden Ansprüche, ferner umfassend ein Spiralelement (60), das um das zweite Ende des Kernelements (12) angeordnet ist.

18. Drahtführung nach Anspruch 17, wobei das Spiralelement (60) eine Platinspirale ist.

19. Drahtführung nach Anspruch 17, wobei das Spiralelement (20) an das Kernelement (12) gelötet ist.

20. Drahtführung nach einem der vorhergehenden Ansprüche, wobei das Kernelement (12) aus einer Formspeicherlegierung zusammengesetzt ist.

21. Drahtführung nach Anspruch 20, wobei das Kernelement (12) aus Nitinol zusammengesetzt ist.

22. Drahtführung nach einem der vorhergehenden Ansprüche, wobei die Vielzahl der Stränge (23) aus rostfreiem Stahl zusammengesetzt ist.

23. Drahtführung nach den Ansprüchen 1 bis 21, wobei die Vielzahl der Stränge (23) aus einer Formspeicherlegierung zusammengesetzt ist.

24. Drahtführung nach Anspruch 23, wobei die Vielzahl der Stränge (23) aus Nitinol zusammengesetzt ist.

25. Drahtführung nach den Ansprüchen 1 bis 21, wobei die Vielzahl der Stränge (23) aus röntgendichtem Material zusammengesetzt ist.

26. Drahtführung nach einem der vorhergehenden Ansprüche, ferner umfassend eine Manschette (42), die das Kernelement (12) und das geflochtene Element (14) umgibt.

27. Drahtführung nach Anspruch 26, wobei die Manschette (42) eine Polyurethanschicht ist, die das Kernelement (12) und das geflochtene Element (14) umgibt.

28. Drahtführung nach einem der vorhergehenden Ansprüche, wobei das geflochtene Element (14) sich um das Kernelement (12) befindet.

29. Drahtführung nach einem der vorhergehenden Ansprüche, wobei das geflochtene Element (14) einen geflochtenen Elementdurchmesser aufweist, der nicht wesentlich größer als ein Kernelementdurchmesser des Kernelements (12) ist.

## Revendications

1. Guide-fil (10) destiné à introduire des dispositifs médicaux dans le corps d'un patient, le guide-fil comprenant :
un élément central (12) ayant une première et une deuxième extrémité ; et
un élément tressé (14) tissé à partir d'une pluralité de brins (23), l'élément tressé (14) étant fixé à l'élément central (12),
l'élément central comportant un épaulement (28) et une hauteur de l'épaulement (28) faisant approximativement l'épaisseur de l'élément tressé (14) pour assurer ainsi une transition en douceur de l'élément central (12) à l'élément tressé (14) ; et
l'élément tressé (14) étant situé autour d'une partie de l'élément central (12), **caractérisé en ce que** l'élément tressé (14) s'étend depuis l'épaulement (28) au-delà de la première extrémité de l'élément central (12) pour constituer une section d'extrémité souple (20) du guide-fil (10).

2. Guide-fil de la revendication 1, dans lequel l'élément tressé (14) est soudé à la première extrémité de l'élément central (12).

3. Guide-fil de la revendication 1, comprenant en outre un adhésif configuré pour attacher l'élément tressé (14) à la première extrémité de l'élément central (12).

4. Guide-fil de l'une quelconque des revendications précédentes, comprenant en outre un revêtement lubrifié (44) entourant l'élément central (12).

5. Guide-fil de la revendication 4, dans lequel le revêtement lubrifié (44) est un revêtement hydrophile.

6. Guide-fil de l'une quelconque des revendications précédentes, dans lequel l'élément central (12) comporte une première section conique (26) proximale de l'élément tressé (14), la première section conique (26) étant configurée pour augmenter la souplesse de l'élément central (12) vers la première extrémité de l'élément central (12).

7. Guide-fil de la revendication 6, dans lequel l'élément central (12) comporte une deuxième section conique (34), configurée pour augmenter la souplesse de l'élément central (12) vers la deuxième extrémité de l'élément central.

8. Guide-fil de la revendication 7, dans lequel l'élément central comporte une section rigide (24) entre la première section conique (26) et la deuxième section conique (34).

9. Guide-fil de la revendication 8, dans lequel la section rigide (24) fait entre environ 50 et 200 cm de longueur.

10. Guide-fil de la revendication 7, dans lequel une distance de la deuxième section conique (34) à la deuxième extrémité de l'élément central (12) fait entre environ 3 et 5 cm.

11. Guide-fil des revendications 6 à 10, dans lequel la première section conique (26) et l'élément tressé (14) constituent une longueur totale d'environ 40 à 80 cm.

12. Guide-fil des revendications 6 à 11, dans lequel la première section conique (26) constitue une longueur d'environ 5 à 15 cm.

13. Guide-fil des revendications 6 à 12, dans lequel une distance de la première section conique (26) à la première extrémité de l'élément central (12) fait entre environ 5 et 15 cm.

14. Guide-fil de l'une quelconque des revendications précédentes, dans lequel l'élément tressé (14) s'étend au-delà de la première extrémité de l'élément central (12) sur une distance comprise entre environ 30 et 50 cm.

15. Guide-fil des revendications 1 à 14, comprenant en outre un élément bobiné (60) s'étendant le long de la longueur de l'élément central (12).

16. Guide-fil de la revendication 15, dans lequel l'élément tressé (14) est attaché à l'élément bobiné (60).

17. Guide-fil de l'une quelconque des revendications précédentes, comprenant en outre un élément bobiné (60) disposé autour de la deuxième extrémité de l'élément central (12).

18. Guide-fil de la revendication 17, dans lequel l'élément bobiné (60) est une bobine de platine.

19. Guide-fil de la revendication 17, dans lequel l'élément bobiné (20) est soudé à l'élément central (12).

20. Guide-fil de l'une quelconque des revendications précédentes, dans lequel l'élément central (12) est composé d'un alliage à mémoire de forme.

21. Guide-fil de la revendication 20, dans lequel l'élément central (12) est composé de Nitinol.

22. Guide-fil de l'une quelconque des revendications précédentes, dans lequel la pluralité de brins (23) est composée d'acier inoxydable.

23. Guide-fil des revendications 1 à 21, dans lequel la pluralité de brins (23) est composée d'un alliage à mémoire de forme.

24. Guide-fil de la revendication 23, dans lequel la pluralité de brins (23) est composée de Nitinol.

25. Guide-fil des revendications 1 à 21, dans lequel la pluralité de brins (23) est composée d'un matériau radio-opaque.

26. Guide-fil selon l'une quelconque des revendications précédentes, comprenant en outre un manchon (42) entourant l'élément central (12) et l'élément tressé (14).

27. Guide-fil de la revendication 26, dans lequel le manchon (42) est une couche de polyuréthane entourant l'élément central (12) et l'élément tressé (14).

28. Guide-fil selon l'une quelconque des revendications précédentes, dans lequel l'élément tressé (14) est situé autour de l'élément central (12).

29. Guide-fil selon l'une quelconque des revendications précédentes, dans lequel l'élément tressé (14) a un diamètre d'élément tressé qui n'est pas sensiblement supérieur à un diamètre d'élément central de l'élément central (12).
